# FASCICULE DE BREVET EUROPEEN

(11) **EP 2 039 341 B1**
(45) Date de publication et mention de la délivrance du brevet: **07.12.2011**
(21) Numéro de dépôt: 08368016.5
(22) Date de dépôt: 12.09.2008
(51) Int. Cl.: A61K 8/49, A61Q 17/00, A61Q 19/08

(54) **Compositions cosmétiques comprenant des dérivés thiazolidines contre les conséquences du stress oxydatif de la peau**
Thiazolidinderivate enthaltende Zusammensetzungen zur Vewendung gegen die Effekte von Oxidationstress der Haut
Cosmetic compositions comprising thiazolidine derivatives for use against the consequences of oxidising stress of the skin

(30) Priorité: 21.09.2007 FR 0706641
(43) Date de publication de la demande: 25.03.2009
(73) Titulaire: Exsymol S.A.M., 98000 Monaco (MC)
(72) Inventeur: Seguin, Marie-Christine, 98000 Monaco (MC)
(74) Mandataire: Macquet, Christophe

(56) Documents cités:
- EP-A- 1 471 067
- FR-A- 2 877 004
- GB-A- 2 088 716

## Description

L'invention concerne des compositions cosmétiques comprenant des dérivés thiazolidine, précisément dans le domaine de la protection de la peau et plus particulièrement dans la lutte contre le stress oxydatif et ses conséquences cutanées.

Par son rôle d'enveloppe externe du corps vis-à-vis de l'atmosphère, la peau est un organe particulièrement sensible à l'environnement auquel elle est soumise. Celui-ci peut être de nature pro-oxydante, dû à des expositions prolongées aux rayonnements ultraviolets du soleil, à des atmosphères enfumées ou contenant des vapeurs de polluants chimiques atmosphériques. Ces "agressions" de nature exogène génèrent en effet un excès d'espèces très réactives, radicalaires ou non, au fort potentiel oxydant. Ce sont notamment des espèces dérivées de l'oxygène moléculaire, souvent appelées espèces oxygénées réactives ou "EOR" ou "ROS" en anglais, telles l'anion superoxyde O2°-, le peroxyde d'hydrogène H2O2, l'oxygène singulet 102, le radical hydroxyle OH°.

Cette surproduction d'espèces réactives EOR est alors responsable de l'altération des constituants biologiques de la peau, en particulier de ceux rencontrés dans les cellules cutanées (ADN, protéines, lipides, glucides). La formation de sous-produits issus de la dégradation de ces molécules biologiques, et en particulier ceux issus des phospholipides membranaires peroxydés par les EOR, est elle aussi néfaste à la peau. Enfin, l'exposition d'une peau à une surproduction d'EOR initie également des réactions biochimiques complexes à l'origine de la production de cytokines pro-inflammatoires, de métabolites mutagènes, et à la mort des cellules cutanées (Briganti S. et al., J. Eur. Acad. Dermatol. Venereol. (2003), vol. 17, pp. 663-669).

D'une manière générale, tous ces phénomènes sont préjudiciables à la peau, plus particulièrement lorsqu'ils atteignent les couches profondes, notamment dans le derme. Il est en outre bien établi aujourd'hui qu'une perte de contrôle par la peau de la présence excessive d'EOR, conduisant à un état communément désigné par « stress oxydatif », est clairement impliquée dans le déclenchement de nombreux désordres ou anomalies cutanés : vieillissement induit, immunosuppression, inflammation/érythème, carcinogenèse, irritation, etc. (Bickers D.R. et al., J. Invest. Dermatol. (2006), vol. 126, pp. 2565-2575 et références citées).

Physiologiquement, et malgré l'existence de systèmes antioxydants naturels de régulation et de protection, enzymatiques (superoxyde dismutases, catalases, peroxydases, etc.) ou non (vitamines A, C, D ou E, caroténoïdes, glutathion, oligo-éléments, etc.), la peau présente, face à un flux important de radicaux libres ou d'espèces EOR, une défense rapidement épuisée et débordée.

Aussi, depuis de nombreuses années, la conception de systèmes capables de limiter les effets délétères de cette surproduction d'espèces réactives, ou même de capturer ou piéger celles-ci, est devenu un sujet de recherche majeur. Ainsi, pour pallier aux insuffisances physiologiques de la peau, de nombreuses molécules, d'origine naturelle (extraits de plantes ou d'animaux terrestres) ou synthétique, ont été développées et proposées à titre de supplément antioxydant ou antiradicalaire, administrées par voie générale (orale) ou par application topique (Darvin M. et al., Skin Pharmacol. Physiol. (2006), vol. 19, pp. 238-247).

Toutefois, il est en général affiché, pour nombre de molécules antioxydantes ou antiradicalaires appartenant à l'état de la technique, une protection efficace à l'encontre des EOR dits de première génération selon l'invention. Par "EOR de première génération", il faut comprendre les espèces dérivées de la réduction de l'oxygène moléculaire (O2°-, H2O2, 102, OH°, ONOO-, etc). Il est par contre plus rarement justifié d'une action vis-à-vis des sous-produits d'oxydation ou produits dits de seconde génération (aldéhydes toxiques, produits finaux issus de la péroxydation lipidique, etc.), eux aussi toxiques et résultant de réactions en chaîne des espèces primaires avec les composants biochimiques de la cellule.

De façon générale, il reste que la sélection d'une substance antioxydante dite "efficace", notamment pour une administration à l'Homme, est difficile. Il est aussi à noter qu'il est souvent avancé un bénéfice in vitro, mais rarement confirmé clairement in vivo (Bickers D.R. et al., J. Invest. Dermatol. (2006), vol. 126, pp. 2565-2575).

En effet, outre la complexité structurale multi-couches de la peau humaine, en conséquence un stress oxydatif s'y exprimant différemment, la substance antioxydante précédemment évoquée doit combiner :
- une efficacité vis-à-vis du plus large panel possible d'espèces oxygénées réactives EOR, en conséquence posséder un large spectre de propriétés "anti-stress",
- une efficacité vis-à-vis des sous-produits toxiques du stress oxydatif,
une réactivité au contact des EOR telle qu'elle ne conduise pas à la formation de produits de réarrangement toxiques,
- une pénétration cutanée (et donc une biodisponibilité) favorable afin de pouvoir atteindre différents sites tissulaires cutanés,
- enfin, une résistance aux systèmes hydrolytiques de la peau (et donc une résistance à sa métabolisation) qui soit garante de son activité in situ.

La présente invention a été développée dans ce contexte de satisfaire à cette demande de produits ou préparations permettant de s'opposer le plus efficacement possible aux effets destructeurs du stress oxydatif et de ses sous-produits toxiques, notamment dans les couches profondes de la peau.

Le choix de la demanderesse s'est ainsi porté sur des dérivés de l'hétérocycle thiazolidine, en particulier sur le 2-oxo-1,3-thiazolidine. Les raisons de cette sélection sont multiples :
En premier lieu, la structure et les propriétés démontrées par le composé 2-oxo-1,3-thiazolidine ont constitué une réponse avantageuse aux différents critères énoncés précédemment, illustrée par :
   - une pénétration cutanée tout à fait favorable, révélée par l'obtention d'une valeur logarithmique de son coefficient de partage ("Log P") comparable à celle obtenue pour des composés reconnus perméants trans-stratum corneum (Arct J. et al., SOFW-J. (2003), vol. 129, pp. 2-9) [test 1 ci-après],
   - une activité antioxydante remarquable au regard de sa capacité à réagir sur un large panel de molécules EOR (constituées par O2°-, H2O2, et OH° produits à l'aide du couple oxydant xanthine oxydase/hypoxanthine) [test 2 ci-après],
   - une capacité insoupçonnée à neutraliser l'espèce électrophile hautement toxique issu de la péroxydation lipidique, l'aldéhyde 4-hydroxynonénal ou "4-HNE" [tests 3 et 4 ci-après].
En second lieu, et c'est un autre aspect important de l'invention, lors d'un suivi de l'hétérocycle 2-oxo-1,3-thiazolidine en milieu aqueux oxydant contenant des EOR, et en particulier le peroxyde d'hydrogène (soit un des EOR les plus toxiques pour les tissus vivants), la demanderesse a découvert, à son étonnement au regard d'une évolution quasi-quantitative et de l'absence de formes oxydées intermédiaires, que cet hétérocycle générait aisément, dès son "placement" sous des conditions oxydatives, le produit d'oxydation ultime, stable, à savoir la taurine [cf. test 5 ci-après]. Un tel comportement est fort utile car il garantit une absence totale de toxicité des sous-produits de réaction du 2-oxo-1,3-thiazolidine avec H2O2, puisque la taurine est en effet un aminoacide naturellement présent dans la peau. Par ailleurs, l'intérêt cutané de former de la taurine se trouve même être aujourd'hui renforcé, puisqu'elle est considérée depuis peu comme osmolyte essentiel dans l'homéostasie de kératinocytes soumis à un stress "UV-induit" (Jancke G. et al., J. Invest. Dermatol. (2003), vol. 121, pp. 354-361).

Le composé 2-oxo-1,3-thiazolidine, en tant que tel, n'est pas un produit nouveau. Dans l'état de la technique, il est à relever sa synthèse à partir d'aminoéthanethiol et de diphényl carbonate, selon un procédé et des conditions particulières qui accordent, d'une manière générale, une réactivité chimique de type phosgène à divers polycarbonates cycliques issus du recyclage de matière plastique (Hata S. et al., J. Appl. Polym. Sci. (2003), vol. 90, pp. 2959-2968). Par contre, et au vu de l'art antérieur porté à la connaissance de la demanderesse, nulle application cosmétique ou dermatologique n'est connue pour spécifiquement cet hétérocycle soufré "2-oxo", ni même une ou plusieurs propriétés vis-à-vis du stress oxydatif pour son analogue "thiono" en position 2 pour lequel, par ailleurs, la demanderesse a aussi observé la formation de taurine en conditions oxydatives [cf. test 5 ci-après].

Il est à signaler toutefois l'existence de l'acide 2-oxothiazolidine-4-carboxylique. Ce composé a fait par contre l'objet, lui, mais aussi certains dérivés esters ou amides, d'intérêts divers et nombreux en cosmétique ou dermatologie : photoprotection (brevets FR 2 877 004 et FR 2 854 160), lutte contre la chute de cheveux (brevet EP 0656 201), agents dépigmentant (brevets US 6,063,389 et US 6,007,827) ou desquamant (brevet FR 2 816 838), etc. A la lecture de sa littérature concernant la protection de la peau d'une manière générale, il n'y est relevé un bénéfice cutané qu'en raison d'une action de stimulation des défenses naturelles des cellules, et tout particulièrement d'une action stimulante sur la synthèse intracellulaire du glutathion. Ce composé n'est pas, de plus, un précurseur de la taurine. Enfin, ce composé est structuralement différent du 2-oxo-1,3-thiazolidine, de sorte que cet enseignement ne présage pas des effets du 2-oxo-1,3-thiazolidine.

Ainsi, selon un premier aspect, et de par cette dimension plurifonctionnelle "anti-stress" représentée par le comportement du composé 2-oxo-1,3-thiazolidine dans un environnement pro-oxydant, tel celui d'une peau soumise à un stress oxydatif, la présente invention s'étend à une composition cosmétique comprenant, en association avec tout excipient physiologiquement compatible avec la peau, et à titre d'ingrédient actif principal, le dérivé de formule générale (I) : 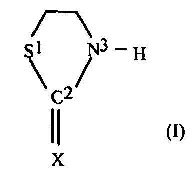 dans laquelle X représente un atome d'oxygène ou de soufre, ledit dérivé ou ladite composition étant destinés à protéger la peau de tout stress générant des radicaux libres ou des espèces réactives de l'oxygène.

Le composé préféré de formule (I) ci-dessus est tel que X représente un atome d'oxygène.

Il est retenu préférentiellement que les compositions susmentionnées sont destinées à protéger la peau contre un stress dont l'origine est choisie parmi les radiations ultraviolettes, la pollution atmosphérique, le contact avec des xénobiotiques chimiques ou des atmosphères enfumées, et tout particulièrement les radiations ultraviolettes. La présente invention vise à prévenir et à lutter contre les signes cutanés résultant de ces stress, notamment contre les différentes manifestations liées au vieillissement de la peau. Elle s'applique particulièrement au domaine de la photoprotection, au regard de :
- l'implication aujourd'hui démontrée du 4-hydroxynonénal ou "4-HNE" dans l'apoptose de cellules soumises à des rayonnements UV-A (Yang Y. et al., J. Biol. Chem. (2003), vol.278, pp.41380-41388), combinée à l'efficacité notable, évoquée ci-avant, du composé 2-oxo-1,3-thiazolidine à piéger cet aldéhyde toxique,
- l'existence rapportée d'un stress "UV-A induit" dans le derme de la peau et dommageable aux constituants protéiniques de la matrice extracellulaire (Wondrak G.T. et al., J. Invest. Dermatol. (2003), vol. 121, pp. 578-586), combinée à la biodisponibilité topique du même composé 2-oxo-1,3-thiazolidine justement dans ces couches dermiques, et en sus de ses caractéristiques intrinsèques : absence de fonctions chimiques ionisables au pH physiologique de la peau, non sensibilité aux hydrolases de la peau, faible poids moléculaire,
- l'évaluation expérimentale menée récemment par la demanderesse dans l'efficacité du composé 2-oxo-1,3-thiazolidine vis-à-vis d'un "stress UV-A induit" ([test 6] ci-après).

Tout particulièrement, ladite composition est destinée à protéger la peau d'un stress "W-induit", et le dérivé de formule générale (I) est tel que X représente un atome d'oxygène.

Avantageusement, la quantité dudit dérivé de formule (I) dans la composition ci-dessus est comprise entre 0,01 et 10 % en poids par rapport au poids total de la composition, de préférence entre 0,05 et 5 % en poids, encore mieux entre 0,5 % et 5 % en poids.

On peut citer, à titre d'exemple d'excipient physiologiquement compatible avec la peau, un tensioactif, un conservateur, un corps gras, un colorant, un émulsionnant, un gélifiant, un émollient, un humectant, un pigment, ou tout autre adjuvant habituellement utilisé en cosmétique.

Selon un mode particulier de l'invention, afin de renforcer l'activité antioxydante ou antiradicalaire, ladite composition est susceptible de comprendre, en outre, un autre élément actif choisi parmi des agents antioxydants, antiradicalaires et/ou anti-pollution couramment sur le marché, de manière telle que l'effet attaché intrinsèquement aux compositions selon l'invention ne soit pas altéré par l'addition envisagée. Ces agents antioxydants, antiradicalaires ou anti-pollution couramment sur le marché peuvent être :
- des vitamines, telles les vitamines C, E, et D,
- des enzymes, telles les superoxydes dismutases, catalases, et peroxydases, d'origine synthétique, végétale, animale, bactérienne ou recombinante par exemple,
- des composés phénoliques, tels des polyphénols (épigallocatéchine-3-gallate) et notamment les flavonoïdes acide férulique, silymarine, génistéine, apigénine, resvératrol.

Toujours de manière facultative, ladite composition susmentionnée peut également associer un filtre solaire UV-A ou B, ou leur mélange, de nature organique (dérivés de la benzophénone) ou inorganique (oxydes métalliques), ou encore un actif cosmétique à effet secondaire, tel un agent hydratant, un agent apaisant, un agent pigmentant, un agent stimulant la synthèse de macromolécules épidermiques et dermiques, et ce, toujours en veillant que ledit actif cosmétique et sa quantité sont présents de manière telle que les propriétés de la composition selon l'invention ne soient pas altérées par l'adjonction envisagée.

Les compositions selon l'invention sont adaptées à une administration par voie topique cutanée, présentées sous toutes les formes normalement utilisées pour une telle administration. De manière avantageuse, elles sont formulées sous forme, par exemple, d'émulsions, crème, lait, gel, lotion, etc.

A titre d'illustration, il est mentionné ci-après quelques exemples de formulation de composition cosmétique selon l'invention, contenant les hétérocycles 2-oxo-1,3-thiazolidine ou 2-thiono-1,3-thiazolidine précités :

**Formule A (crème)**

| | |
|---|---|
| 2-oxo-1,3-thiazolidine | 1 % |
| Polyisobutène hydrogéné | 7 % |
| Myristate d'isobutyle | 3 % |
| Palmitate de cétyle | 7 % |
| Monostéarate d'éthylène glycol | 5 % |
| Laurate sorbitan | 2 % |
| Polysorbate 20 | 2 % |
| Carbomer (copolymère d'acrylate/acrylamide & huile minérale) | 0,3 % |
| Phénoxyéthanol | 0,5 % |
| Eau | qsp 100 % |

**Formule B (gel)**

| | |
|---|---|
| 2-thiono-1,3-thiazolidine | 0,5 % |
| Carbomer (copolymère d'acrylate/acrylamide & huile minérale) 1,5 % | |
| Benzoate de sodium | 0,2 % |
| Acide sorbique | 1 % |
| 1,3-butanediol | 10 % |
| Glycérine | 5 % |
| Soude | 0,13 % |
| Phénoxyéthanol | 0,9 % |
| Eau | qsp 100 % |

L'invention est illustrée ci-après, à titre purement indicatif par les tests suivants évoqués plus haut dans la description de l'invention (tests 1 à 6). Il est aussi à signaler que les premiers résultats d'études in vivo menées sur l'Homme sont également en mesure d'illustrer l'utilisation des dérivés thiazolidines aux fins de la présente invention.

### Test 1 : coefficient de partage du composé 2-oxo-1,3-thiazolidine

Les valeurs de coefficient de partage n-octanol/eau (Log P) ont été obtenus expérimentalement conformément au protocole décrit dans la ligne directrice n° 107 de l'OCDE (adoptée le 27/7/1995) pour les essais de produits chimiques.

Le log P du composé 2-oxo-1,3-thiazolidine selon l'invention est comparé à celui obtenu pour la caféine et l'éthanol (tableau 1 ci-après).

**Tableau 1**

| Composé | Log P |
|---|---|
| éthanol | - 0,31 |
| caféine | - 0,07 |
| 2-oxo-1,3-thiazolidine | - 0,27 |

Le résultat obtenu pour le 2-oxo-1,3-thiazolidine est comparable à celui obtenu pour les composés reconnus perméants trans-stratum corneum.

### Test 2 : mise en évidence de l'effet antioxydant du composé 2-oxo-1,3-thiazolidine

### a) mesure de l'activité piégeur du radical hydroxyle (OH°) :

La méthode, décrite par Rehman A. et coll. (British J. Pharmacol. (1997), vol. 122, pp. 1702-1706), est utilisée pour la détermination de la constante de vitesse de piégeage du radical hydroxyle [Ks(OH°)], le composé 2-oxo-1,3-thiazolidine selon l'invention étant comparé à une molécule de référence, la taurine.

Expérimentalement, la substance testée est dissoute dans un milieu tamponné à pH 7,4 auquel est ajouté un milieu générateur d'OH°. Après une heure d'incubation à 37°C, la réaction est arrêtée à l'aide d'acide trichloroacétique. Après addition du révélateur colorimétrique, l'acide thiobarbiturique, il est mesuré l'absorbance à 532 nm pour différentes concentrations, puis calculé le Ks(OH°) relatif à chacune des substances. Les résultats sont reportés dans le tableau 2a ci-après.

**Tableau 2a**

| Composé | Ks(OH°) (109.M-1.s-1) |
|---|---|
| taurine | 5 ,32 |
| 2-oxo-1,3-thiazolidine | 13,52 |

Les résultats, rassemblant les valeurs moyennes obtenues à partir de cinq expérimentations indépendantes, soulignent une capacité à piéger le radical hydroxyle (OH°), pour le composé 2-oxo-1,3-thiazolidine, très largement supérieure à celle de la taurine, supérieure même à l'acide ascorbique (vitamine C) pour lequel la littérature annonce un Ks(OH°) de 10,1 Giga.M-1.s-1 (Cabelli D.E., J. Phys. Chem. (1983), vol. 87, pp. 1809-1812).

### b) mesure du pouvoir antioxydant "global" :

Le système de production d'EOR, décrit par Nowak D. et coll. (Biomed. Biochem. Acta (1991), vol. 50, pp. 265-272) et mettant en jeu le couple xanthine oxydase/hypoxanthine, est utilisé pour la détermination du pouvoir antioxydant "global". Celui-ci est caractérisé par la somme des effets à l'encontre de l'anion superoxyde O2°-, du peroxyde d'hydrogène H2O2, et du radical hydroxyle OH°, et est exprimé en suivant le pourcentage de protection d'une molécule "détectrice", le désoxyribose.

Le composé 2-oxo-1,3-thiazolidine selon l'invention est également comparé à la taurine. Expérimentalement, la substance testée est dissoute dans un milieu tamponné à pH 7,5, additionné de NaCl, MgCl2 et CaCl2. Après leur incubation à 37°C en présence du couple xanthine oxydase/hypoxanthine, d'EDTA, et de désoxyribose, la réaction est arrêtée à l'aide d'acide trichloroacétique. Après addition d'acide thiobarbiturique, il est réalisé une mesure de densité optique à 532 nm, exprimée par la suite en pourcentage de protection du désoxyribose (après comparaison avec un contrôle). Les résultats sont reportés dans le tableau 2b ci-après

**Tableau 2b**

| Composé | % de protection |
|---|---|
| taurine (20 mM) | 19 |
| 2-oxo-1,3-thiazolidine (10 mM) | 19 |
| 2-oxo-1,3-thiazolidine (20 mM) | 25 |

Pour le composé 2-oxo-1,3-thiazolidine, il est ainsi observé une protection du désoxyribose supérieure à celle obtenue avec la taurine (protection identique pour une concentration deux fois moindre).

### Test 3 : mise en évidence de la capacité du composé 2-oxo-1,3-thiazolidine à neutraliser la cytotoxicité du 4-hydroxynonénal

L'étude expérimentale a été réalisée sur une lignée fibroblastique adhérente de hamster appelée "V79", maintenue en atmosphère humide à 37 °C et 5 % de CO2, puis ensemencée dans des plaques 96 puits à raison de 0,5.104 cellules par puit. Les cellules sont alors soumises à un état de stress, toxique, avec le remplacement du milieu de culture par un milieu contenant du 4-hydroxynonénal (ou 4-HNE) à la concentration de 6 ppm, milieu auquel est ajouté simultanément le composé 2-oxo-1,3-thiazolidine selon l'invention.

La viabilité cellulaire des fibroblastes est mesurée par la méthode d'incorporation au bromure de 3-(4,5-diméthylthiazol-2-yl)-2,5-diphenyltétrazolium (ou "MTT"). Les résultats, rassemblant les valeurs moyennes obtenues à partir de trois expérimentations indépendantes, sont présentés dans le tableau 3 ci-après, en comparaison avec ceux obtenus pour la N-acétyl-cystéine à 2 mM choisie comme molécule de référence positive (restauration totale d'une viabilité cellulaire par rapport à un contrôle et des cellules non objets d'un stress 4-HNE).

**Tableau 3**

| | % viabilité cellulaire |
|---|---|
| contrôle | 100 |
| HNE 6 ppm | 34 |
| N-acétyl-cystéine 2 mM | 126 |
| 2-oxo-1,3-thiazolidine 0,1 mM | 40 |
| 2-oxo-1,3-thiazolidine 1 mM | 58 |
| 2-oxo-1,3-thiazolidine 2,5 mM | 100 |

Les résultats du tableau 3 soulignent, pour le composé 2-oxo-1,3-thiazolidine selon l'invention, une viabilité cellulaire dose-dépendante, ainsi qu'une capacité identique à celle de la référence à protéger les cellules soumises à un stress 4-HNE.

### Test 4 : mise en évidence de l'effet anti-clastogène du composé 2-oxo-1,3-thiazolidine en présence d'une concentration génotoxique de 4-hydroxynonénal

L'étude a été réalisée selon des conditions expérimentales (ensemencement) identiques à celles présentées dans le test 3 ci-avant, avec une exposition des cellules à un état de "stress 4-HNE" à la concentration génotoxique de 1 ppm, décrite (Eckel P.M., Molecular Aspects of Medecine (2003), vol. 24, pp. 161-165) comme entraînant des cassures de l'ADN cellulaire et la formation de micronoyaux (effet clastogène).

Le nombre de cellules à micronoyaux est compté après lecture des lames au microscope à épifluorescence, sur 2000 cellules et après marquage à l'acridine orange (colorant de l'ADN).

Les résultats, rassemblant les valeurs moyennes obtenues à partir de deux expérimentations indépendantes, sont présentés dans le tableau 4 ci-après, en comparaison avec ceux obtenus pour la N-acétyl-cystéine à 2 mM choisie comme molécule de référence positive (nombre de cellules à micronoyaux voisin de celui du "contrôle" ou témoin non exposé).

**Tableau 4**

| | Nbre de cellules à micronoyaux/2000 cellules |
|---|---|
| contrôle | 19 |
| stress-HNE 1 ppm | 58 |
| N-acétyl-cystéine 2 mM | 27 |
| 2-oxo-1,3-thiazolidine 5 mM | 21 |

Il est ainsi observé, pour le composé 2-oxo-1,3-thiazolidine selon l'invention, une capacité à prévenir l'apparition de cellules à micronoyaux induite par un stress génotoxique 4-HNE.

### Test 5 : mise en évidence "in tubo" de la formation de taurine au contact d'une solution d'H2O2

A une solution aqueuse à 10 % en dérivés 2-oxo-1,3-thiazolidine ou 2-thiono-1,3-thiazolidine selon l'invention, tamponnée à pH 7,4 et à 37 °C, sont ajoutés 4 équivalents d'une solution de peroxyde d'hydrogène (H2O2), soit un faible excès d'espèces oxydantes par rapport aux 3 équivalents théoriques nécessaires à une conversion complète (100 %) en taurine. L'évolution du mélange réactionnel est alors suivie par chromatographie en phase liquide (HPLC muni d'un détecteur UV à 254 nm), conduisant aux résultats exposés dans le tableau 5 ci-après et à la mise en évidence d'une formation quasi-exclusive et quantitative de taurine après réaction des dérivés 2-oxo-1,3-thiazolidine ou 2-thiono-1,3-thiazolidine au contact de H2O2.

**Tableau 5**

| | taurine (% formation à partir du 2-oxo-1,3-thiazolidine) | taurine (% formation à partir du 2-thiono-1,3-thiazolidine) |
|---|---|---|
| 3h | 38 | 30 |
| 7h | 56 | 50 |
| 24h | 82 | 77 |
| 48h | 93 | 89 |

### Test 6 : mise en évidence de l'effet protecteur du composé 2-oxo-1,3-thiazolidine en présence d'un stress "UV-A induit"

L'étude a été réalisée selon des conditions expérimentales (ensemencement) identiques à celles présentées dans les tests 3 et 4 ci-avant, mais avec une exposition des cellules V79 à des rayonnements ultra-violets (λ 315-400 nm) à la dose modérée de 5 J.cm-2 (stress "UV-A-induit").

La formation d'EOR intracellulaire est mesurée à l'aide d'une sonde fluorescente perméante "CMDCF" dérivée de la fluorescéine (10 µm, 15 minutes d'incubation).

Les pourcentages de cellules CMDCF dites "positives" (cellules avec formation intracellulaire de ROS) sont présentés dans le tableau 6 ci-après, en comparaison à des cellules non irradiées, puis à des cellules traitées avec la N-acétyl-cystéine (témoin de référence) et différentes concentrations du composé 2-oxo-1,3-thiazolidine.

**Tableau 6**

| | % cellules CMDCF positives |
|---|---|
| Cellules non irradiées | 3 |
| Cellules irradiées | 30 |
| Cellules irradiées + N-acétyl-cystéine 5 mM | 4 |
| Cellules irradiées + 2-oxo-1,3-thiazolidine 0,625 mM | 24,7 |
| Cellules irradiées + 2-oxo-1,3-thiazolidine 1,25 mM | 15,3 |
| Cellules irradiées + 2-oxo-1,3-thiazolidine 2,5 mM | 12 |
| Cellules irradiées + 2-oxo-1,3-thiazolidine 5 mM | 7,3 |

Le composé 2-oxo-1,3-thiazolidine selon l'invention présente une capacité dose-dépendante à protéger les cellules soumises à un stress UVA-induit, et similaire au témoin de référence.

## Revendications

1. Composition cosmétique destinée à protéger la peau de tout stress générant des radicaux libres ou des espèces réactives de l'oxygène, comprenant, en association avec tout excipient physiologiquement compatible avec la peau, et à titre d'ingrédient actif principal, un dérivé de formule (I) : 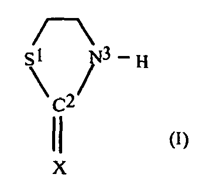 dans laquelle X représente un atome d'oxygène ou de soufre.

2. Composition cosmétique selon la revendication 1, **caractérisée en ce que** le dérivé de formule (I) est tel que X représente un atome d'oxygène.

3. Composition cosmétique selon l'une des revendications 1 et 2, **caractérisée en ce que** le dérivé de formule (I) est présent dans une quantité comprise entre 0,01 et 10 % en poids par rapport au poids total de la composition.

4. Composition cosmétique selon la revendication 3, **caractérisée en ce que** la quantité dudit composé de formule (I) est comprise entre 0,05 et 5 % en poids par rapport au poids total de la composition.

5. Composition cosmétique selon l'une quelconque des revendications 1 à 4, **caractérisée en ce qu'**elle comprend, en outre, un autre élément actif antioxydant, antiradicalaire ou anti-pollution.

6. Composition cosmétique selon la revendication 5, **caractérisée en ce que** ledit autre élément actif est choisi parmi des vitamines, des enzymes, des composés phénoliques.

7. Composition cosmétique selon l'une quelconque des revendications 1 à 6, **caractérisée en ce qu'**elle peut associer un filtre solaire UV-A ou B, organique ou inorganique, ou leur mélange, ou encore un actif cosmétique à effet secondaire, tel un agent hydratant, un agent apaisant, un agent pigmentant, un agent stimulant la synthèse de macromolécules épidermiques et dermiques.

8. Composition cosmétique selon l'une quelconque des revendications 1 à 7, **caractérisée en ce qu'**elle est formulée sous forme d'émulsions, crème, lait, gel, lotion.

9. Composition cosmétique selon l'une quelconque des revendications 1 à 8, **caractérisée en ce que** le stress est choisi parmi des radiations ultraviolettes, de la pollution atmosphérique, du contact avec des xénobiotiques chimiques ou des atmosphères enfumées.

10. Composition cosmétique selon la revendication 9, **caractérisée en ce que** le stress est des radiations ultraviolettes.

## Claims

1. Cosmetic composition intended for protecting skin against any stress generating free radicals or reactive oxygen species, comprising, in association with any physiologically compatible excipient with skin, and as main active ingredient, a derivative of formula (I) : 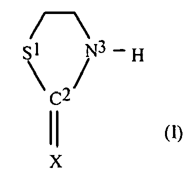 in which X represents an oxygen or sulphur atom.

2. Cosmetic composition according to claim 1, **characterized in that** the derivative of formula (I) is such that X represents an oxygen atom.

3. Cosmetic composition according to one of claims 1 and 2, **characterized in that** the derivative of formula (I) is present in a quantity comprised between 0.01 and 10 % in weight in relation to total weight of the composition.

4. Cosmetic composition according to claim 3, **characterized in that** the quantity of said derivative of formula (I) is comprised between 0.05 and 5 % in weight in relation to total weight of the composition.

5. Cosmetic composition according to any of claims 1 to 4, **characterized in that** it furthermore comprises another antioxidant, antiradical or anti-pollution active element.

6. Cosmetic composition according to claim 5, **characterized in that** said other active element is chosen among vitamins, enzymes, phenolic compounds.

7. Cosmetic composition according to any of claims 1 to 6, **characterized in that** it can associate an organic or inorganic UV-A or B sunscreen or their mixture, or else a cosmetic active ingredient with a secondary effect such as a moisturizing agent, a soothing agent, a pigmenting agent, an agent stimulating the synthesis of epidermic and dermic macromolecules.

8. Cosmetic composition according to any of claims 1 to 7, **characterized in that** it is formulated under the form of emulsions, cream, milk, gel, lotion.

9. Cosmetic composition according to any of claims 1 to 8, **characterized in that** the stress is chosen among ultraviolet radiation, atmospheric pollution, contact with chemical xenobiotics or smoky atmospheres.

10. Cosmetic composition according to claim 9, **characterized in that** the stress is ultraviolet radiation.

## Patentansprüche

1. Kosmetische Zusammensetzung um die Haut vor jeglicher Beanspruchung, die freie Radikale oder reaktive Sauerstoffspezies erzeugt, zu schützen, umfassend, in Verbindung mit einem beliebigen physiologisch hautverträglichen Hilfsstoff als Hauptwirkstoff, ein Derivat der folgenden Formel (I): 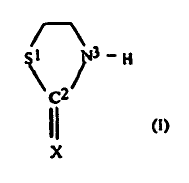 in welcher X ein Sauerstoffatom oder Schwefelatom darstellt.

2. Kosmetische Zusammensetzung nach Anspruch 1, **dadurch gekennzeichnet, dass** im Derivat der Formel (I), X ein Sauerstoffatom darstellt.

3. Kosmetische Zusammensetzung nach einem der Ansprüche 1 und 2, **dadurch gekennzeichnet, dass** der Anteil des Derivates der Formel (I) zwischen 0,01 und 10 Gew.% bezogen auf das Gesamtgewicht der Zusammensetzung liegt.

4. Kosmetische Zusammensetzung nach Anspruch 3, **dadurch gekennzeichnet, dass** der Anteil der Verbindung der Formel (I) zwischen 0.05 und 5 Gew.% bezogen auf das Gesamtgewicht der Zusammensetzung liegt.

5. Kosmetische Zusammensetzung nach einem der Ansprüche 1 bis 4, **dadurch gekennzeichnet, dass** sie außerdem ein anderes aktives antioxidatives, antiradikales oder umweltfreundliches Element beinhaltet.

6. Kosmetische Zusammensetzung nach Anspruch 5, **dadurch gekennzeichnet, dass** besagtes aktives Element aus Vitaminen, Enzymen, phenolischen Verbindungen ausgewählt wird.

7. Kosmetische Zusammensetzung nach einem der Ansprüche 1 bis 6, **dadurch gekennzeichnet, dass** sie einen organischen oder anorganischen, UV-A oder B Sonnenschutzfilter, oder eine Mischung davon, bzw. einen kosmetischen wirkstoff mit Nebeneffekten, wie ein feuchthaltendes Mittel, ein schmerzlinderndes Mittel, ein Pigmentierungsmittel, und ein Mittel zur Stimulation der Synthese von epidermalen und dermalen Makromolekülen, verknüpfen kann.

8. Kosmetische Zusammensetzung nach einem der Ansprüche 1 bis 7, **dadurch gekennzeichnet, dass** sie in Form einer Emulsion, Creme, Milch, Gel, Lotion formuliert wird.

9. Kosmetische Zusammensetzung nach einem der Ansprüche 1 bis 8, **dadurch gekennzeichnet, dass** die Beanspruchung aus durch UV Strahlung, Luftschadstoffen, dem Kontakt mit xenobiotischen Chemikalien oder verrauchtem Umfeld ausgewählt ist.

10. Kosmetische Zusammensetzung nach Anspruch 9, **dadurch gekennzeichnet, dass** die Beanspruchung aus UV Strahlung besteht.
